(19) Europäisches Patentamt
European Patent Office
Office européen des brevets

(11) Veröffentlichungsnummer : **0 347 677 B1**

(12) **EUROPÄISCHE PATENTSCHRIFT**

(45) Veröffentlichungstag der Patentschrift :
**04.03.92 Patentblatt 92/10**

(51) Int. Cl.$^5$ : **C07C 41/22,** C07C 43/225, C07D 317/52

(21) Anmeldenummer : **89110527.2**

(22) Anmeldetag : **10.06.89**

# (54) Verfahren zur Herstellung von Aryloxychlormethanen.

(30) Priorität : **23.06.88 DE 3821130**

(43) Veröffentlichungstag der Anmeldung :
**27.12.89 Patentblatt 89/52**

(45) Bekanntmachung des Hinweises auf die Patenterteilung :
**04.03.92 Patentblatt 92/10**

(84) Benannte Vertragsstaaten :
**DE FR GB IT**

(56) Entgegenhaltungen :
**EP-A- 0 197 868**
**DE-C- 1 183 096**

(56) Entgegenhaltungen :
**CHEMISTRY AND INDUSTRY, UK Chemical Industry In 1977, Nr. 1, 1. JUnner 1977 ROBERT LOUW et al. "Selective side-chain chlorination of methoxybenzenes" Seiten 127-128**

(73) Patentinhaber : **BAYER AG**
**W-5090 Leverkusen 1 Bayerwerk (DE)**

(72) Erfinder : **Mayr, Herbert, Prof.-Dr.**
**Meisenweg 1**
**W-2401 Gross-Grönau (DE)**
Erfinder : **Cambanis, Anton, Dr.**
**Laakstrasse 426**
**W-4670 Lünen (DE)**
Erfinder : **Bäuml, Engelbert, Dr.**
**An der Münze 18**
**W-2401 Gross-Grönau (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 347 677 B1

## Beschreibung

Die vorliegende Erfindung betrifft ein Verfahren zur Herstellung von Aryloxychlormethanen aus nicht oder unvollständig chlorierten Aryloxymethanen.

Aryloxychlormethane sind bekannte Verbindungen, die als Zwischenprodukte für organische Synthesen benötigt werden [s. z. B. DE-OS 35 02 106 und Chem. Ber. 121, 339 - 345 (1988)].

Bisher sind folgende Verfahren zur Herstellung von Aryloxychlormethanen bekannt geworden:

Bis(aryloxy)chlormethane [siehe Formel (II), $R_1$ = H, $R_2$ = $Ar_2O$], können durch Erhitzen von Bis(aryloxy)acetylchloriden im Vakuum [s. Ber. Deut. Chem. Ges. 68, 2151 (1935) und J. Prakt. Chem. 7, 70 (1958)] oder durch Behandeln von Tris(aryloxy)methanen mit Acetylchlorid oder Trichloracetylchlorid [ s. Chem. Ber. 95, 1859 (1962) und Rec. Trav. Chim. 92, 11 (1973)] sowie durch Umsetzung von Bis(aryloxy)essigsäuren mit Thionylchlorid [s. J. Prak. Chem. 320, 128 (1978)] erhalten werden. Bis(aryloxy)dichlormethane [s. Formel (II), $R_1$ = Cl, $R_2$ = $Ar_2O$] können aus Diarylcarbonaten durch Erhitzen mit Phosphorpentachlorid [s. Chem. Ber. 94, 544 (1961) und J. Heterocycl. Chem. 19, 1205 (1982)] erhalten werden. 2,2-Dichlor-1,3-benzodioxol [s. Formel (II), $R_1$ = Cl, $Ar_1O$ und $R_2$ = gemeinsam o-Phenylendioxy] ist hergestellt worden durch Reaktion von 1,3-Benzodioxol mit Phosphorpentachlorid [s. Chem. Ber. 94, 544 (1961) und 96, 1382 (1963)]. Schließlich wurden zur Herstellung von Aryloxytrichlormethanen [s. Formel (II), $R_1$ = $R_2$ = Cl] die entsprechenden Phenole mit Thiophosgen zur Arylchlorthioformiaten umgesetzt und diese chloriert [s. J. Gen. Chem. USSR 28, 2539 (1958) zitiert in J. Org. Chem. 37, 2651 (1972)].

Nachteilig bei diesen Verfahren ist, daß Ausgangsprodukte, insbesondere Bis(aryloxy)essigsäurederivate, schwer zugänglich sind, bei der Herstellung der Ausgangsprodukte, insbesondere bei der Herstellung von Diarylcarbonaten, das nur mit großem Sicherheitsaufwand handzuhabende Phosgen benötigt wird, bei den Umsetzungen unerwünschte Nebenprodukte entstehen, die besonderen Aufwand für ihre Abtrennung erfordern, insbesondere beim aus Phosphorpentachlorid entstehendem Phosphoroxichlorid, und Umsetzungen mit Thiophosgen ebenfalls großen sicherheitstechnischen Aufwand erfordern.

Aus Chemistry and Industry vom 5. Februar 1977, S. 127 - 128 ist bekannt, daß man Anisol radikalisch zu in der Seitenkette mono-, di- und/oder tri-substituierten Produkten chlorieren kann. In Konkurrenz zu dieser Reaktion erfolgt der Eintritt von Chlor in den aromatischen Kern. Welche dieser Reaktionen bevorzugt stattfindet und ob eine selektive Seitenkettenchlorierung möglich ist, kann nicht vorhergesehen werden (s. z. B. a. a. O. S. 127, linke Spalte, Zeilen 1 - 3 und S. 128, linke Spalte, Zeilen 4 - 17). Dies gilt insbesondere dann, wenn man statt Anisolen Aryloximethane anderer Struktur einsetzt.

Es wurde nun ein Verfahren zur Herstellung von Aryloxychlormethanen gefunden, das dadurch gekennzeichnet ist, daß man nicht oder unvollständig chlorierte Aryloxymethane der Formel (I)

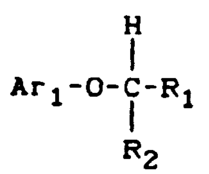

(I),

in der

$Ar_1$ für einen gegebenenfalls substituierten Arylrest mit 6 bis 10 C-Atomen,

$R_1$ für Wasserstoff oder Chlor und

$R_2$ für eine $Ar_2$-O-Gruppe steht, wobei $Ar_2$ einen gegebenenfalls substituierten Arylrest mit 6 bis 10 C-Atomen bedeutet, der gleich oder verschieden von $Ar_1$ sein kann

und wobei die Gruppierung $Ar_1O$ gemeinsam mit $R_2$ und dem dazwischen befindlichen C-Atom auch für einen gegebenenfalls substituierten Rest der Formel (Ia)

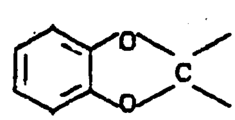

(Ia)

stehen kann,

einer radikalisch initiierten Chlorierung unterwirft.

Vorzugsweise steht $Ar_1$ für Phenyl, einfach oder mehrfach, z. B. 1- bis 5-fach halogeniertes Phenyl oder

für Phenyl, das mit einem $NO_2$-, CN-, $CCl_3$- oder $CF_3$-Rest substituiert ist.

Vorzugsweise steht $R_1$ für Wasserstoff.

Vorzugsweise steht $R_2$ für eine $Ar_2$-O-Gruppe, in der $Ar_2$ für Phenyl, einfach oder mehrfach, z. B. 1- bis 5-fach halogeniertes Phenyl oder für Phenyl, das mit einem $NO_2$-, CN-, $CCl_3$- oder $CF_3$-Rest substituiert ist, steht.

Weiterhin ist es bevorzugt, daß die Gruppierung $Ar_1O$ gemeinsam mit $R_2$ und dem dazwischen befindlichen C-Atom für einen gegebenenfalls substituierten Rest der Formel (Ia) steht.

Als Halogensubstituenten für Phenylreste und den Rest der Formel (Ia) sind Fluor und Chlor bevorzugt.

Ausgangsprodukte der Formel (I) sind bekannte Verbindungen (s. z. B. E. V. Dehmlow und J. Schmidt, Tetrahedron Letters 1976, 95) oder entstehen im erfindungsgemäßen Verfahren als Zwischenprodukte.

Als Chlorierungsmittel kommen beispielsweise elementares Chlor oder Sulfurylchlorid in Frage. Besonders wirtschaftlich ist es, die erfindungsgemäße Chlorierung $^1$H-NMR-spektroskopisch zu verfolgen und so lange Chlorierungsmittel einzuleiten, bis der größtmöglichste Anteil des gewünschten Produkts im Reaktionsgemisch vorliegt. Die Chlorierungsmittel können auch im Überschuß angewendet werden, insbesondere, wenn man Produkte herstellen möchte, die am Methan-Kohlenstoffatom kein Wasserstoffatom mehr enthalten.

Um in der erfindungsgemäßen Chlorierung Radikale zu initiieren, können beispielsweise geringe Mengen von üblichen Radikalbildnern zugesetzt werden. Geeignet sind beispielsweise Zusätze von Azo-bis-isobutyronitril, beispielsweise in Mengen von 0,0001 bis 0,05 Mol pro Mol eingesetztes Aryloxymethan. Man kann Radikale auch durch Bestrahlung initiieren, beispielsweise durch Bestrahlung mit einer UV-Lampe.

Geeignete Temperaturen für die erfindungsgemäße Chlorierung sind beispielsweise solche zwischen 0 und 100°C. Beim Zusatz von Radikalbildnern (z. B. Azo-bis-isobutyronitril) ist es bevorzugt, bei relativ höheren Temperaturen zu arbeiten, z. B. bei 60 bis 100°C. Bei der Initiierung von Radikalen durch Bestrahlung ist es bevorzugt, bei relativ niedrigen Temperaturen zu arbeiten, z. B. bei 10 bis 40°C.

Im allgemeinen ist es vorteilhaft, das erfindungsgemäße Verfahren in Gegenwart eines inerten Lösungsmittels durchzuführen. Beispielsweise kommen hierfür chlorierte Kohlenwasserstoffe infrage, insbesondere perchlorierte Kohlenwasserstoffe. Bevorzugt wird Tetrachlorkohlenstoff verwendet.

Weiterhin ist es im allgemeinen vorteilhaft, das erfindungsgemäße Verfahren unter weitgehendem bis vollständigem Ausschluß von Feuchtigkeit und Sauerstoff durchzuführen, beispielsweise indem man getrocknete Lösungsmittel verwendet und unter einer Stickstoffatmosphäre arbeitet.

Das nach der erfindungsgemäßen Umsetzung vorliegende Reaktionsgemisch kann beispielsweise aufgearbeitet werden, indem man es einer fraktionierten Destillation im Vakuum unterwirft und gegebenenfalls vorhandenes Lösungsmittel vorher abzieht.

Auf die erfindungsgemäße Weise sind Aryloxychlormethane der Formel (II) erhältlich,

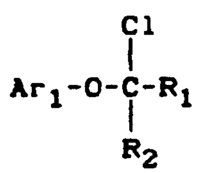

(II),

in der

$Ar_1$, $R_1$ und $R_2$ die bei Formel (I) angegebene Bedeutung haben.

Das erfindungsgemäße Verfahren ist ganz besonders geeignet zur Herstellung von Verbindungen der Formel (II), bei denen $Ar_1$ für Phenyl, einfach oder mehrfach, z. B. 1- bis 5-fach halogeniertes Phenyl oder für Phenyl, das mit einem $NO_2$-, CN-, $CCl_3$- oder $CF_3$-Rest substituiert ist,

$R_1$ für Wasserstoff oder Chlor und $R_2$ für eine $Ar_2$-O-Gruppe, in der $Ar_2$ für Phenyl, einfach oder mehrfach, z. B. 1- bis 5-fach halogeniertes Phenyl oder für Phenyl, das mit einem $NO_2$-, CN-, $CCl_3$- oder $CF_3$-Rest substituiert ist oder die Gruppierung $Ar_1O$ gemeinsam mit $R_2$ und dem dazwischen befindlichen C-Atom für einen gegebenenfalls substituierten Rest der Formel (Ia) steht.

Mit dem erfindungsgemäßen Verfahren gelingt die Herstellung von Aryloxychlormethanen der Formel (II) im allgemeinen in Ausbeuten zwischen 70 und 98 % d. Th. Dies ist ausgesprochen überraschend, da die zahlreichen bekannten Verfahren zur Herstellung von Aryloxychlormethanen entweder die radikalisch initiierte Chlorierung vermeiden, offenbar weil (auch) eine Chlorierung des Aromatenteils des Moleküls befürchtet wurde, oder, soweit sie eine radikalisch initiierte Chlorierung umfassen, nur die Herstellung von Aryloxichlormethanen mit von der Formel (II) abweichenden Strukturen betreffen. Das erfindungsgemäße Verfahren geht von gut und einfach zugänglichen Ausgangsprodukten aus, liefert bei der Verwendung von Chlor oder Sulfurylchlorid als Chlorierungsmittel nur gasförmig entweichende, aus dem Chlorierungsmittel entstehende Nebenprodukte und erfordert keinen besonderen sicherheitstechnischen Aufwand.

Beispiele

Allgemeine Arbeitsweise:

A. In ein Gemisch aus 50 ml trockenem Tetrachlorkohlenstoff, 30 mMol einer Verbindung der Formel (I) und 0,45 mMol Azo-bis-isobutyronitril wurde in einer mit Stickstoff gespülten Apparatur bei 80 bis 85°C elementares Chlor eingeleitet, bis nach $^1$H-NMR-spektroskopischer Kontrolle der gewünschte Chlorierungsgrad erreicht war. Dann wurde Tetrachlorkohlenstoff bei vermindertem Druck abgezogen und der Rückstand im Vakuum destilliert.

B. Ein Gemisch aus 30 ml trockenem Tetrachlorkohlenstoff, 20 mMol einer Verbindung der Formel (I), Sulfurylchlorid in einer Menge von 2 Äquivalenten pro Äquivalent durch Chlor zu ersetzenden Wasserstoff und 0,30 mMol Azo-bis-isobutyronitril wurde in einer mit Stickstoff gespülten Apparatur am Rückfluß gekocht, bis nach $^1$H-NMR-spektroskopischer Kontrolle der gewünschte Chlorierungsgrad erreicht war. Dann wurde Tetrachlorkohlenstoff bei vermindertem Druck abgezogen und der Rückstand im Vakuum destilliert.

Die Einzelheiten der nach diesen allgemeinen Arbeitsweisen durchgeführten Versuche sind aus der folgenden Tabelle 1 ersichtlich:

**Tabelle 1**

| Bsp. Nr. | Einsatzmaterial der Formel (I) | | | Arbeitsweise | Reaktionszeit (Std. | erhaltenes Produkt der Formel (II) | | | Ausbeute (% d. Th.) | Siedepunkt (°C/ mbar) | Schmelzpunkt (°C) |
|---|---|---|---|---|---|---|---|---|---|---|---|
| | $Ar_1$ | $R_1$ | $R_2$ | | | $Ar_1$ | $R_1$ | $R_2$ | | | |
| 1 | $C_6H_5$ | H | $C_6H_5O$ | A | 0,5 | $C_6H_5$ | H | $C_6H_5O$ | 72 | 120-122 / 0.1 | |
| 2 | $pClC_6H_4$ | H | $pClC_6H_4O$ | A | 0,5 | $pClC_6H_4$ | H | $pClC_6H_4O$ | 73 | 145-150 / 0.1 | 65-67,5 (Pentan) |
| 3 | $pClC_6H_4$ | H | $pClC_6H_4O$ | B | 1,5 | $pClC_6H_4$ | H | $pClC_6H_4O$ | 73 | 145-150 / 0.1 | 65-67,5 (Pentan) |
| 4 | $C_6H_5$ | H | $C_6H_5O$ | A | 2 | $C_6H_5$ | Cl | $C_6H_5O$ | 95 | 100-105 / 0.07 | 40-42 |
| 5 | $pClC_6H_4$ | H | $pClC_6H_4O$ | A | 2 | $pClC_6H_4$ | Cl | $pClC_6H_4O$ | 96 | 170-175 / 0.4 | 30,5-31,5 (Pentan) |
| 6 | $pClC_6H_4$ | H | $pClC_6H_4O$ | B | 4,5 | $pClC_6H_4$ | Cl | $pClC_6H_4O$ | 96 | 170-175 / 0.4 | |
| 7 | 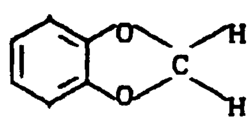 | | | A | 3,5 | 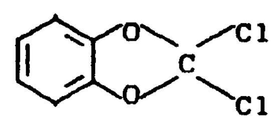 | | | 95 | 83-86 / 20 | |

Beispiel 8

Es wurde verfahren wie in Beispiel 6, jedoch wurde kein Azo-bis-isobutyronitril zugesetzt, Radikale durch Bestrahlung mit einer UV-Lampe (300 nm) initiiert und bei 22°C gearbeitet. Es wurde mit einer Ausbeute von 95 % d. Th. ein Produkt erhalten, das identisch war mit dem in Beispiel 6 erhaltenen Produkt.

## Patentansprüche

1. Verfahren zur Herstellung von Aryloxychlormethanen, dadurch gekennzeichnet, daß man nicht oder unvollständig chlorierte Aryloxymethane der Formel (I)

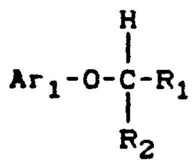

(I),

in der

$Ar_1$ für einen gegebenenfalls substituierten Arylrest mit 6 bis 10 C-Atomen,

$R_1$ für Wasserstoff oder Chlor und

$R_2$ für eine $Ar_2$-O-Gruppe steht, wobei $Ar_2$ einen gegebenenfalls substituierten Arylrest mit 6 bis 10 C-Atomen bedeutet, der gleich oder verschieden von $Ar_1$ sein kann

und wobei die Gruppierung $Ar_1O$ gemeinsam mit $R_2$ und dem dazwischen befindlichen C-Atom auch für einen gegebenenfalls substituierten Rest der Formel (Ia)

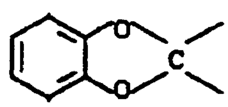

(Ia)

stehen kann,

einer radikalisch initiierten Chlorierung unterwirft.

2. Verfahren nach Anspruch 1, dadurch gekennzeichnet, daß in Formel (I) $Ar_1$ für Phenyl, einfach oder mehrfach halogeniertes Phenyl oder für Phenyl, das mit einem $NO_2$-, CN-, $CCl_3$- oder $CF_3$-Reste substituiert ist, $R_1$ für Wasserstoff und $R_2$ für eine $Ar_2$-O-Gruppe, in der $Ar_2$ für Phenyl, einfach oder mehrfach halogeniertes Phenyl oder für Phenyl, das mit einem $NO_2$-, CN-, $CCl_3$- oder $CF_3$-Rest substituiert ist, steht.

3. Verfahren nach Ansprüchen 1 bis 2, dadurch gekennzeichnet, daß man als Chlorierungsmittel elementares Chlor oder Sulfurylchlorid einsetzt.

4. Verfahren nach Ansprüchen 1 bis 3, dadurch gekennzeichnet, daß man Radikale durch Zusatz von Radikalbildnern oder durch Bestrahlung initiiert.

5. Verfahren nach Ansprüchen 1 bis 4, dadurch gekennzeichnet, daß man es unter weitgehendem bis vollständigem Ausschluß von Feuchtigkeit und Sauerstoff durchführt.

6. Verfahren nach Ansprüchen 1 bis 5, dadurch gekennzeichnet, daß man es bei 0 bis 100°C durchführt.

7. Verfahren nach Ansprüchen 1 bis 6, dadurch gekennzeichnet, daß man es in Gegenwart eines inerten Lösungsmittels durchführt.

8. Verfahren nach Ansprüchen 1 bis 7, dadurch gekennzeichnet, daß man das nach der Chlorierung vorliegende Reaktionsgemisch einer fraktionierten Destillation im Vakuum unterwirft und gegebenenfalls vorhandenes Lösungsmittel vorher abzieht.

## Claims

1. Process for the preparation of aryloxychloromethanes, characterized in that unchlorinated or incompletely chlorinated aryloxymethanes of the formula (I)

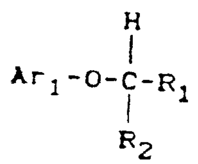

(I)

in which

$Ar_1$ represents an optionally substituted aryl radical having 6 to 10 C atoms,

$R_1$ represents hydrogen or chlorine and

$R_2$ represents an $Ar_2$-O- group, where $Ar_2$ denotes an optionally substituted aryl radical having 6 to 10 C atoms, which may be identical to or different from $Ar_1$

and where the grouping $Ar_1O$ together with $R_2$ and the C atom situated in between may also represent an optionally substituted radical of the formula (Ia)

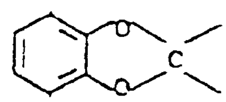

(Ia)

are subjected to a radical-initiated chlorination.

2. Process according to Claim 1, characterized in that, in formula (I), $Ar_1$ represents phenyl, monohalogenated or polyhalogenated phenyl or represents phenyl which is substituted by an $NO_2$, CN, $CCl_3$ or $CF_3$ radical, $R_1$ represents hydrogen and $R_2$ represents an $Ar_2$-O- group in which $Ar_2$ represents phenyl, monohalogenated or polyhalogenated phenyl or represents phenyl which is substituted by an $NO_2$, CN, $CCl_3$ or $CF_3$ radical.

3. Process according to Claims 1 to 2, characterized in that elemental chlorine or sulphuryl chloride is employed as the chlorinating agent.

4. Process according to Claims 1 to 3, characterized in that radicals are initiated by addition of radical promoters or by irradiation.

5. Process according to Claims 1 to 4, characterized in that it is carried out with extensive to complete exclusion of moisture and oxygen.

6. Process according to Claims 1 to 5, characterized in that it is carried out at 0 to 100°C.

7. Process according to Claims 1 to 6, characterized in that it is carried out in the presence of an inert solvent.

8. Process according to Claims 1 to 7, characterized in that the reaction mixture present after the chlorination is subjected to a fractional distillation in vacuo and, if desired, solvent present is stripped off beforehand.

## Revendications

1. Procédé de préparation d'aryloxychlorométhanes, caractérisé en ce qu'on soumet à une chloration, amorcée par des radicaux, des aryloxyméthanes non chlorés ou incomplètement chlorés de formule (I)

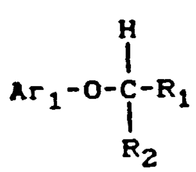

(I),

dans laquelle

$Ar_1$ représente un reste aryle ayant 6 à 10 atomes de carbone, éventuellement substitué,

$R_1$ est l'hydrogène ou le chlore et

$R_2$ représente un groupe $Ar_2$-O, dans lequel $Ar_2$ est un reste aryle ayant 6 à 10 atomes de carbone éventuellement substitué, qui peut être identique à $Ar_1$ ou peut en être différent

et le groupement $Ar_1O$ peut former conjointement avec $R_2$ et avec l'atome de carbone qui se trouve entre eux un reste éventuellement substitué de formule (Ia)

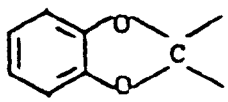

(Ia)

2. Procédé suivant la revendication 1, caractérisé en ce que dans la formule (I) $Ar_1$ représente un groupe phényle, un groupe phényle une ou plusieurs fois halogéné ou un groupe phényle qui est substitué avec un reste $NO_2$, CN, $CCl_3$ ou $CF_3$, $R_1$ représente l'hydrogène et $R_2$ est un groupe $Ar_2$-O dans lequel $Ar_2$ est un reste phényle, phényle halogéné une ou plusieurs fois ou phényle qui est substitué avec un reste $NO_2$, CN, $CCl_3$ ou $CF_3$.

3. Procédé suivant les revendications 1 et 2, caractérisé en ce qu'on utilise comme agent de chloration le chlore élémentaire ou le chlorure de sulfuryle.

4. Procédé suivant les revendications 1 à 3, caractérisé en ce qu'on amorce la formation de radicaux par l'addition de générateurs de radicaux ou par irradiation.

5. Procédé suivant les revendications 1 à 4, caractérisé en ce qu'il est mis en oeuvre en l'absence notable ou totale d'humidité et d'oxygène.

6. Procédé suivant les revendications 1 à 5, caractérisé en ce qu'il est mis en oeuvre à une température de 0 à 100°C.

7. Procédé suivant les revendications 1 à 6, caractérisé en ce qu'il est mis en oeuvre en présence d'un solvant inerte.

8. Procédé suivant les revendications 1 à 7, caractérisé en ce qu'on soumet à une distillation fractionnée sous vide le mélange réactionnel obtenu après la choration et on chasse au préalable le solvant éventuellement présent.